(19) Europäisches Patentamt
European Patent Office
Office européen des brevets

(11) Publication number: **0 309 765 A2**

# (12) EUROPEAN PATENT APPLICATION

(21) Application number: 88114355.6

(22) Date of filing: 02.09.88

(51) Int. Cl.[4]: A61K 31/50

(30) Priority: 30.09.87 US 102703

(43) Date of publication of application: 05.04.89 Bulletin 89/14

(84) Designated Contracting States: AT BE CH DE ES FR GB IT LI LU NL SE

(71) Applicant: PENNWALT CORPORATION
Pennwalt Building Three Parkway
Philadelphia Pennsylvania 19102(US)

(72) Inventor: Radov, Lesley Ann
28 Henderson Drive
Penfield New York 14526(US)
Inventor: Thomas, Telfer Lawson
17 Candlewood Drive
Pittsford New York 14534(US)

(74) Representative: Kraus, Walter, Dr. et al
Patentanwälte Kraus, Weisert & Partner
Thomas-Wimmer-Ring 15
D-8000 München 22(DE)

## (54) 1(2H)-Phthalazinones as cytoprotective agents.

(57) 1(2H)-Phthalazinones such as trans-6-[2-(4-methoxyphenyl)ethenyl]-2-(3-aminopropyl)phthalazin-1(2H)-one hydrobromide, trans-6-[2-(4-methoxyphenyl)ethenyl]-2-[3-(dimethylamino)propyl]phthalazin-1(2H)-one hydrobromide, and trans-6-[2-(4-hydroxphenyl)ethenyl]-2-[3-(dimethylamino)propyl]phthalazin-1(2H)-one hydrochloride are useful as cytoprotective agents in mammals.

EP 0 309 765 A2

# 1(2H)-Phthalazinones as Cytoprotective Agents

## Background of the Invention

A well known untoward side effect of anti-inflammatory agents such as aspirin and inodmethacin is gastrointestinal ulceration due to irritation of the gastric mucosa. The multifunctional basis of ulcerogenisis, however, has made it difficult to cure. Within the last several years, the focus of anti-ulcer therapy has shifted away from the development of anti-secretory agents (such as cimetidine), and towards the development of a new class of 'cytoprotective' agents.

These types of agents have a protective effect on the gastric mucosa, unrelated to inhibition of acid secretion. It has been discovered that, unlike cimetidine, certain 1(2H)-phthalazinones exhibit indomethacin-induced gastric lesions and, therefore, represent a novel class of cytoprotective agents which are expected to be useful for the treatment of gastrointestinal diseases such as ulcers. These phthalazinones were disclsed in U.S. patent 4,665,181 as anti-inflammatory agents useful for the treatment of arthritis in warm blooded animals.

## Summary of the Invention

The present invention is a method of cytoprotective treatment in a mammal which comprises administering, to a warm blooded animal in need of such treatment, a 1(2H)-pthalazinone compound of the formula I.

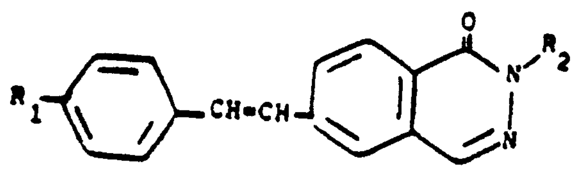

(I)

including all stereoisomeric forms and pharmaceutically acceptable addition salts thereof,
wherein
$R_1$ is hydrogen, hydroxyl or $C_1$-$C_4$ alkoxy,
$R_2$ is hydrogen, $(CH_2)_mOH$,

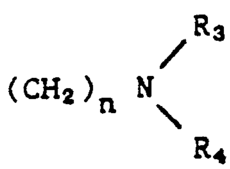 , 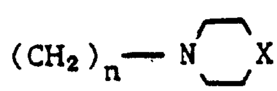, 1-methyl-4-piperidinyl

or 1-pyrrolidinyl,

$R_3$ and $R_4$ are independently hydrogen or $C_1$-$C_8$ alkyl,
X is $CH_2$ or $NR_5$,
$R_5$ is hydrogen or $C_1$-$C_8$ alkyl,
m is 2 or 3,
and n is 2-4,
provided that when m is 2, $R_1$ is hydrogen.

These compounds have been found to possess cytoprotective properties in warm blooded animals and thus are expected to be useful as anti-ulcer agents in humans.

Included in the amine radicals represented by

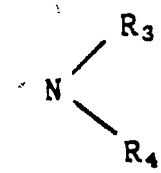

are amino, methylamino, ethylamino, dimethylamino, diethylamino, and diisopropyl amino. Included in the amine radicals represented by

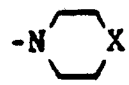

are piperidino, piperazino, and 4-methylpiperazino.

The preferred 1(2H)-phthalazinones of formula I are those wherein, $R_1$ is hydrogen or methoxy, and $R_2$ is 3-(dimethylamino)propyl, 3-(diisopropylamino)propyl, 2-(dimethylamino)ethyl, 4-(dimethylamino)butyl, 3-aminopropyl, 3-piperidinopropyl, 3-(1-piperazinyl)propyl, or 1-methyl-4-piperidinyl, and the stereoisomeric form is the trans isomer. By trans it is meant that the hydrogen atoms of the ethenylene moiety are on opposite sides of the plane of the ethenylene double bond.

The addition salts may be with acids or bases. The preferred salts are acid addition salts of inorganic acids such as hydrogen chloride, hydrogen bromide, sulfuric acid, or organic acids such as acetic, lactic, maleic, fumaric, malic, succinic, tartaric and methanesulfonic acids.

An additional invention is trans-6-[2-(4-methoxyphenyl) ethenyl]-2-[3-(1-piperazino]propyl]phthalazin-1-(2H)-one.

## Methods of Preparation

Methods for preparing compounds of Formula I and appropriate intermediates are disclosed in U.S. patent 4,665,181. In addition an alternative route to the compounds of Formula I is shown in Scheme I.

Scheme I

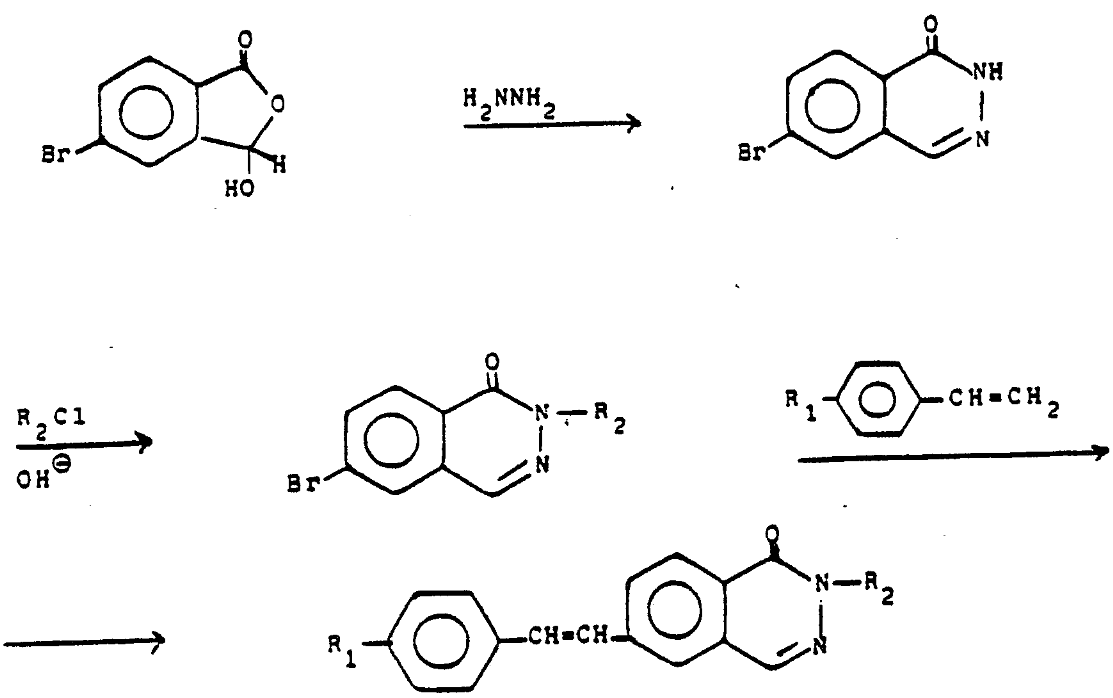

5-Bromo-3-hydroxyphthalide is reacted in alcoholic solution with hydrazine to give 6-bromophthalazin-1-(2H)-one which in turn is reacted with the appropriate (dialkylamino)alkylhalide in a polar solvent such as DMSO in the presence of a base such as KOH to give the 2-(dialkylaminoalkyl)-6-bromophthalazin-1(2H)-one. the styryl group is then attached by reacting the 6-bromo-2-substituted-phthalazin-1(2H)-one with styrene or 4-methoxystyrene in solvents such as DMSO or acetonitrile or mixtures thereof, in the presence of the reagents tri(o-tolyl)phosphine and palladium acetate to give the 6-[2-(phenyl or 4-methoxyphenyl)-ethenyl]-2-[(dialkylamino)alkyl]phthalazin-1(2H)-one. Specific methods for preparing the compounds of this invention are disclosed in the Examples.

## Formulations

For pharmaceutical purposes, the compounds of this invention can be administered to warm-blooded animals perorally or parenterally as active ingredients in customary dosage unit compositions. These compositions consist essentially of a dosage unit form containing the active ingredient and at least one inert pharmaceutical carrier. Dosage unit forms contemplated by the present invention include tablets, capsules, solutions, suspensions, lozenges, coated pills and parenteral compositions such as intramuscular, intravenous or intradermal preparations. Sustained release dosage forms are also contemplated where the active ingredient is bound to an ion exchange resin which, optionally, can be coated with a diffusion barrier coating to modify the release properties of the resin.

The quantity of active ingredient administered in such dosage forms can vary over a wide range depending upon the mode of administration, the size and weight of the patient and whether the nature of the treatment is prophylactic or therapeutic in nature. In general, dosage unit forms contain from about 5 mg to 100 mg of the active ingredient and in man the dose is administered from 1 to 4 times daily. The total daily dosage will be from about 5 mg to 500 mg although lower and higher amounts can be used. A preferred total daily dose would be from 10 mg to 100 mg of active ingredient.

## Biological Data

The methodology for determining the degree of inhibition of gastric lesion formation by the compounds of formula 1 is described below and the results, expressed as percent inhibition, are given in Table 1.

### Reversal of Indomethacin-induced Gastric Lesions

Male, Sprague-Dawley rats weighing 200-225 grams were fasted 24 hours prior to the intraperitoneal administration of the test compounds. The standard screening dosed employed was either 100 or 50 mg/kg. One hour later, the rats were orally administered 25 mg/kg of indomethacin. Both indomethacin and the compounds were suspended in 1% Clearjel ("Instant Clearjel®", a food grade pregelatinized starch from National Starch and Chemical Corporation, New York, N.Y.) or .85% NaCl saline for dosing. Six hours after indomethacin administration, the animals were sacrificed, their stomachs removed, opened along the line of greater curvature, and rinsed with distilled water. The number of macroscopically visible hemmorhagic lesions (dark brown discolorations) in the glandular portion of the stomach was enumerated.

The results obtained with the title compounds of Examples 1-17 are given in Table 1.

Table 1

Effect of Intraperitoneal Administration of Compounds

| Example No. | Dose (mg/kg) | Percent Inhibition** |
|---|---|---|
| 1 | 100 | 92 |
| 2 | 50 | 91 |
| 3 | 100 | 65 |
| 4 | 50 | 7 |
| 5 | 50 | 89 |
| 6 | 100 | 63 |
| 7 | 50 | 79 |
| 8 | 50 | 96 |
| 9 | 50 | 85 |
| 10 | 50 | 61 |
| 11 | 100 | 45 |
| 12 | 100 | 43 |
| 13 | 50 | 99 |
| 14 | 50 | 96.3 |
| 15 | 100 | 7.2 |
| 16 | 50 | 80 |
| 17 | 100 | 59 |

**Percent Inhibition is equal to:

$$\frac{[(\text{No. of control group lesions})-(\text{No. of test group lesions})]}{[\text{No. of control group lesions}]} \times 10$$

where the control group animals received saline solution one hour prior to receiving indomethacin.

The preferred compound for cytoprotective activity is considered to be trans 6-[2-(4-methoxyphenyl)-ethenyl]-2-(3-aminopropyl)phthalazinone-1(2H)-one hydrobromide.

## EXAMPLES

Example 1

Trans-6-[2-(4-methoxyphenyl)ethenyl]-2-[2-(diisopropylamino)-ethyl]phthalazin-1(2H)-one hydrobromide

Step a

6-Bromophthalazin-1(2H)-one

5-Bromo-3-hydroxyphthalide (refer to U.S. patent (4,665,181) (49 gm) was slurried in 200 ml isopropanol. Hydrazine (20 ml) was added and the mixture refluxed for 2 hours. The reaction mixture was allowed to cool and the product filtered off, washed with isopropanol and dried. The product weighed 46.8 gm and melted at 280-283 °C.

Step b

6-Bromo-2-[2-(diisopropylamino)ethyl]phthalazin-1(2H)-one

6-Bromophthalazinone (11.2 gm, 0.05 mole) was slurried in 80 ml dimethylsulfoxide. To this was added 22 ml 45% KOH (0.25 mole). After stirring 10 minutes, 16 gm 2-(diisopropylamino)ethyl chloride hydrochloride was added and the mixture stirred for 18 hours. A water bath was used to moderate the slightly exothermic reaction (temperature < = 25 °C). Water (100 ml) was added to the reaction mixture, which was then stirred another hour.

The product was filtered, washed to colorless washings with water and dried. The product weighed = 17.7 gm. Thin layer chromatography (TLC; 40% ethyl acetate in cyclohexane on an ammonia treated silica plate) showed a single product $R_f$ = 0.59; no starting material material, $R_f$ = 0.23, was visible. Two barely visibly trace spots, $R_f$ = 0.36 and 0.32 were also present.

Recrystallization of a 6.3 gm sample from 35 ml methoxyethanol yielded 3.8 gm of product showing a single spot on TLC.

Step c

Trans-6-[2-(4-methoxyphenyl)ethenyl]-2-[2-diisopropylamino)-ethyl]phthalazin-1(2H)-one hydrobromide

6-Bromo-2-[2-(diisopropylamino)ethyl]phthalazin-1(2H)-one (10.6 gm, 0.03 mole, prepared in Step b) was stirred into 16 ml DMSO plus 16 ml acetonitrile. To the resulting mixture was added 4.5 gm 4-methoxystyrene (0.033 mole), 0.1 gm tri(o-tolyl)phosphine, and 0.02 gm palladium acetate. The reaction mixture was stirred at reflux (95-100 °C) for 24 hours, allowed to cool to about 60 °C and diluted with 60 ml of isopropanol. It was then stirred for an hour, filtered, washed to colorless washings with ispropanol and dried. The product weighed 11.3 gm. After recrystallization from 80 ml 1:1::ethanol: water, the product weighed 10.0 gm and melted at 251-252 °C.

Example 2

Trans-6-[2-(4-methoxyphenyl)ethenyl]-2-(3-piperidinopropyl)-phthalazin-1(2H)-one hydrobromide

6-Bromo-2-(3-piperidinopropyl)phthalazin-1(2H)-one (7.2 gm, 0.018 mole) (prepared as in Example 1, except that 1-(3-chloropropyl)-piperidine hydrochloride was substituted for the 2-(diisopropylamino)ethyl chloride hydrochloride) was stirred into 10 ml DMSO plus 10 ml acetonitrile. To the resulting mixture was added 2.8 gm 4-methoxystyrene (0.021 mole), 0.1 gm tri(o-tolyl)phosphine, and 0.02 gm palladium acetate.

The reaction mixture was stirred at reflux (95-100° C) for 24 hours, allowed to cool to about 60° C and diluted with 40 ml of ispropanol. It was then stirred for an hour, filtered, washed to colorless washings with isopropanol and dried. The product weighed 8.6 gm. After recrystallization from 50 ml methoxyethanol the product weighed 6.4 gm and melted at 248-250° C.

Example 3

Trans-6-[2-(4-methoxyphenyl)ethenyl]-2-[2-(dimethylamino)-ethyl]phthalazin-1(2H)-one hydrobromide

6-Bromo-2-[2-(dimethylamino)ethyl]phthalazin-1(2H)-one (7.2 gm, 0.018 mole) (prepared as in Example 1, except that 2-(dimethylamino)ethyl chloride hydrochloride was substituted for the 2-(diisopropylamino)-ethyl chloride hydrochloride) was stirred into 16 ml DMSO plus 16 ml acetonitrile. To the resulting mixture was added 4.5 gm 4-methoxy styrene (0.033 mole), 0.1 gm tri(o-tolyl)-phosphine, and 0.02 gm palladium acetate. The reaction mixture was stirred at reflux (95-100° C) for 24 hours, allowed to cool to about 60° C and diluted with 60 ml of isopropanol. It was then stirred for an hour, filtered, washed to colorless washings with isopropanol and dried. The product weighed 10.7 gm. After recrystallization from 240 ml methoxyethanol the product weighed 6.4 gm and melted at 265-267° C.

Example 4

Trans-6-[2-(4-methoxyphenyl)ethenyl]-2-(2-morpholinoethyl)-phthalazin-1(2H)-one hydrobromide

6-Bromo-2-(2-morpholinoethyl)phthalazin-1(2H)-one (10.1 gm, 0.03 mole) (prepared as in Example 1, except that 2-morpholinoethyl chloride hydrochloride was substituted for the 2-(diisopropylamino)ethyl chloride hydrochloride) was stirred into 16 ml DMSO plus 16 ml acetonitrile. To the resulting mixture was added 4.5 gm 4-methoxystyrene (0.033 mole), 0.1 gm tri(o-tolyl)phosphine, and 0.02 gm palladium acetate. The reaction mixture was stirred at reflux (95-100° C) for 24 hours, allowed to cool to about 60° C and diluted with 60 ml of isopropanol. It was then stirred for an hour, filtered, washed to colorless washings with isopropanol and dried. The product weighed 11.9 gm. After recrystallization from 40 ml ethanol plus 30 ml water the product weighed 9.2 gm and melted at 254-256° C.

Example 5

Trans-6-[2-(4-methoxyphenyl)ethenyl]-2-[3-(1-piperazino)-propyl]phthalazin-1(2H)-one

6-Bromo-2-(3-piperazinopropyl)phthalazin-1(2H)-one (2.1 gm, 0.0062 mole) (prepared as in Example 1, except that 3-piperazinopropyl chloride hydrochloride was substituted for the 2-(diisopropylaminoethyl chloride hydrochloride) was stirred into 4 ml DMSO plus 4 ml acetonitrile. To the resulting mixture was added 1 gm 4-methoxystyrene (0.0075 mole), 0.05 gm tri(o-tolyl)phosphine, and 0.01 gm palladium acetate. The reaction mixture was stirred at reflux (95-100° C) for 24 hours, allowed to cool to about 60° C and diluted with 20 ml of isopropanol. It was then stirred for 2 hours, filtered, washed to colorless washings with isopropanol and dried. The product weighed 1.4 gm. and melted at 206-209° C.

Example 6

Trans-6-[2-(4-methoxyphenyl)ethenyl]-2-(1-methyl-4-piperidinyl)phthalazin-1(2H)-one

1-Methyl-4-hydrazinopiperidine (41.2 gm) was dissolved in 1000 ml of ethanol. To this was added 90 gm trans-5-[2-(4-methoxyphenyl)ethenyl]-3-hydroxyphthalide (refer to U.S. patent 4,665,181). The resulting mixture was refluxed for 5 hours, then concentrated to dryness and the product taken up in 2000 ml of 2.5

normal HCl and extracted three times with 700 ml ethyl acetate. The product was basified, extracted into chloroform, and concentrated to dryness. The product was dissolved in isopropanol, acidified with HCl, heated to reflux, charcoaled and allowed to cool and crystallize. After three more crystallizations 3.7 gm of pure material was obtained melting at 297 to 298 °C.

Example 7

Trans-6-[2-(4-methoxyphenyl)ethenyl]-2-[3-(methylamino)-propyl]phthalazin-1(2H)-one hydrobromide

Trans-6-[2-(4-Methoxyphenyl)ethenyl]-2-[3-(dimethylamino)propyl]phthalazin-1(2H)-one (8.9 gm, 0.024 mole) (refer to U.S. patent 4,665,181) in 150 ml toluene was treated with 27 ml 12% phosgene in toluene and stirred for 7 days. The solvent was removed and the residue recrystallized from 25 ml carbon tetrachloride plus 15 ml toluene, yielding 5.4 gm of the chlorocarbonyl intermediate.

The intermediate was heated in 125 ml water at about 90 °C for 30 minutes. The solution was clarified, basified with potassium carbonate and extracted into ethyl acetate. The solvent was removed and the residue dissolved in 30 ml ethanol and acidified with 3 ml 30% HBr in acetic acid. The product was allowed to crystallize, filtered and washed with isopropanol. The purified product weighed 3.8 gm and melted at 256-258 °C.

Example 8

Trans-6-[2-(4-methoxyphenyl)ethenyl]-2-(3-aminopropyl)-phthalazin-1(2H)-one hydrobromide

Step a

6-Bromo-2-(3-carboxypropyl)phthalazin-1(2H)-one

To a slurry of 56 gm 6-bromophthalazin-1(2H)-one (0.25 mole) in 600 ml DMSO was added 110 ml 45% KOH followed by 58.5 gm ethyl 4-bromobutyrate. The temperature of the mildly exothermic reaction was moderated with a water bath (ca. 25 °C) and was stirred for 20 hours. Ethanol (750 ml) was added, then the mixture was acidified with 125 ml concentrated HCl, diluted with 2500 ml water over about 30 minutes, stirred an additional 30 minutes, filtered, washed with water and isopropanol and dried. The product weighed 63.6 g. A tlc of the product showed product, Rf = 0.43 plus a trace of starting material, Rf = 0.63.

Step b

6-Bromo-2-(3-aminopropyl)phthalazin-1(2H)-one

6-Bromo-2-(3-carboxypropyl)phthalazin-1(2H)-one (9.3 gm, 0.03 mole), was dissolved in 185 ml acetone by adding 4.7 ml triethylamine (0.0335 mole). The slightly cloudy solution was clarified, cooled to < 10 °C and 3.8 gm methyl chloroformate (0.04 mole) was added over about 5 minutes. It was stirred with cooling for another 30 minutes, and then combined with a solution of 4 gm sodium azide (0.062 mole) in 25 ml water. The resulting solution was stirred for 30 minutes, added to 400 ml ice water and extracted three times with 80 ml methylene dichloride. This solution was dried, 4 ml of trifluoroacetic acid was added, and the solution was refluxed for 18 hours. Methanol (100 ml) was added and the solution shaken with dilute potassium carbonate solution to free the base. The resulting solution was acidified with 10 ml of 30% HBr in acetic acid, diluted with 900 ml ether, filtered and washed with ethyl acetate. The product weighed 6.3 gm and showed a single spot on tlc, Rf = 0.28 (using 10% methanol in chloroform on an ammonia treated silica plate).

Step c

6-Bromo-2-[3-[(t-butoxycarbonyl)amino)propyl]phthalazin-1(2H)-one

6-Bromo-2-(3-aminopropyl)phthalazin-1(2H)-one (13.6 gm) was dissolved in a mixture of 130 ml chloroform and 60 ml t-butanol. Triethylamine (5.8 ml) and 9.1 gm of di(t-butyl) dicarbonate were added and the solution was allowed to stand for 5 days in the dark. The solution was added to a solution of 15 gm citric acid in 380 ml water and stirred for a few minutes. The chloroform layer was separated and the aqueous layer extracted twice more with 75 ml chloroform. The combined chloroform extracts were extracted twice with 75 ml water and once with 75 ml potassium carbonate solution. The solution was dried, the solvent removed and the residue slurried in 110 ml of cyclohexane for 1 hour. The product was filtered off and washed with cyclohexane. It weighed 12.7 gm and melted at 130-132° C.

Step d

Trans-6-[2-(4-methoxyphenyl)ethenyl]-2-[3-aminopropyl]-phthalazin-1(2H)-one hydrobromide

A mixture of 9.1 gm 6-bromo-2-[3-(t-butoxycarbonyl)-amino]propyl]phthalazin-1(2H)-one (0.0238 mole, prepared as described above), 3.7 ml 4-methoxystyrene (0.028 mole), 18 ml DMSO, 18 ml acetonitrile, 3 gm triethylamine (0.03 mole), 0.15 gm tri-(o-tolyl)phosphine (0.0005 mole) and 0.03 gm palladium acetate (0.00013 mole) were refluxed together for 20 hours. It was diluted with 40 ml isopropanol after allowing to cool to about 80° C. The solution was added to 200 ml water and 200 ml ethyl acetate, shaken well, and the organic layer separated. The water layer was extracted twice more with 100 ml ethyl acetate, and the organic solution was washed with water, dried and concentrated to dryness. The crude product (weight = 0.3 gm) was recrystallized from 45 ml isopropanol, yielding 6.6 gm of pure product having an $R_f$ of 0.26 using 40% ethyl acetate in cyclohexane on a silica plate.

The protecting group was removed by slurrying in 130 ml isopropanol containing 160 ml 30% HBr in acetic acid. The product was filtered off and washed with isopropanol. It weighed 5.1 gm and showed a single spot on tlc. After recrystallization from 65 ml 95% ethanol plus 10 ml water it weighed 3.9 gm and melted at 291-293° C.

Example 9

Trans-6-[2-(4-methoxyphenyl)ethenyl]-2-[4-(dimethylamino)-butyl]phthalazin-1(2H)-one hydrobromide

Step a

6-Bromo-2-[4-(dimethylamino)butyl]phthalazin-1(2H)-one

6-Bromo-2-(3-carboxypropyl)phthalazin-1(2H)-one (18.4 gm 0.06 mole, prepared in Example 8 was dissolved in a mixture of 400 ml methylene dichloride and 10 ml triethylamine. Methyl chloroformate (7.6 gm, 0.07 mole) was added, the mixture was stirred for a few minutes, and then the solvent was removed on a rotary evaporator. The residue was dissolved in 30 ml absolute ethanol and 1.2 gm sodium borohydride (0.03 mole) was added portionwise over 1 hour. The mixture was stirred for 20 hours, diluted with 20 ml water, stirred 15 minutes, then basified with 25 ml water containing 5 ml 45% potassium hydroxide and stirred 20 hours. The solution was acidified and the product filtered off, washed with water and dried. The product weighed 7.1 gm. Work up of the filtrates allowed recovery of 9.1 gm of starting acid. .bp [BP?]

The alcohol above (7.1 gm, 0.024 mole) was dissolved in 75 ml pyridine and stirred with 8.6 gm p-toluenesulfonyl chloride for 6 hours at ambient temperature. The mixture was added to 225 gm ice plus 125 ml concentrated HCl and the product extracted into methylene dichloride. Removal of the solvent left 7.5 gm solid. This material was dissolved in 45 ml DMSO and 8 ml dimethylamine. After heating at 80° C for 6

hours the mixture was allowed to cool and stand over night. It was added to 300 ml water and extracted three times with 150 ml ethyl acetate, washing each extract three times with 75 ml water. The product was then extracted from the organic solution with three 100 ml portions of dilute HCl. The acidic solution was basified, the product extracted into chloroform, dried and the solvent removed, leaving 3.1 gm of crystalline product.

Step b

Trans-6-[2-(4-methoxyphenyl)ethenyl]-2-[4-dimethylamino)-butyl]-phthalazin-1(2H)-one hydrobromide

The procedure used was identical to that used in preparing 6-[2-(4-methoxyphenyl)ethenyl]-2-[2-(dimethylamino)-ethyl]phthalazin-1(2H)-one (Example 3 above), except that 6-bromo-2-[4-(dimethylamino)-butyl]phthalazin-1(2H)-one was substituted for 6-bromo-2[2-(dimethylamino)ethyl]phthalazin-1(2H)-one. The weight of product obtained from 7.5 gm starting halide was 7.1 gm after recrystallization from 50 ml of methoxyethanol. It melted at 225-227° C.

Example 10

Cis-6-[2-(4-methoxyphenyl)ethenyl]-2-[3-(dimethylamino)propyl]phthalazin-1(2H)-one cyclohexylsulfamate

Trans-6-[2-(4-methoxyphenyl)ethenyl]-2-[3-(dimethylamino)propyl]-phthalazin-1(2H)-one (48 gm) in 7000 ml 20% isopropanol in ethanol was irradiated in a 12 liter flask with a 450 watt Hanovia ultraviolet lamp for 8 hours. The solution was concentrated to dryness and the residue slurried in 55 ml ethanol, heated to a cloudy solution, allowed to cool and filtered off the transisomer that had crystallized from the solution. The filtrate, which was cisisomer contaminated with a trace of transisomer and some minor impurities, was diluted with water, basified and the product extracted into chloroform. The solution was concentrated to dryness and the residue chromatographed on a preparative liquid chromatographic apparatus using a silica column. The product was eluted with a 5:3:1:1 mixture of ethyl acetate:n-butanol:acetic acid:water giving 12.7 gm of oily product. The product was converted to the cyclohexylsulfamic acid salt using 10 gm of the acid in 70 ml of acetone. it weighed 11.6 gm and melted at about 110-120° C.

Example 11

Trans-6-[2-(4-Hydroxyphenyl)ethenyl]-phthalazin-1(2H)-one

Trans-6-[2-(4-methoxyphenyl)ethenyl]-1(2H)-phthalazinone (24.3 g) was heated with 100 g anhydrous pyridine hydrochloride at 160° C (bath temp. 180° C) with stirring for about 4 hours, then poured while hot into 1000 ml water, stirred for 15 minutes, filtered, and washed with water, isopropanol and diethyl ether. Dry weight = 19 g. Recrystallized from DMF (3 ml/g). Wt = 14 g; m.p. 323-324° C.

Example 12

Trans-6-[2-phenylethenyl]phthalazin-1(2H)-one

The procedure used was identical to that used in preparing 6-[2-(4-methoxyphenyl)ethenyl]-2-[2-(dimethylamino)-ethyl]phthalazin-1(2H)-one (Example 3 above), except that 6-bromophthalazin-1(2H)-one was substituted for 6-bromo-2-[2-(dimethylamino)ethyl]phthalazin-1(2H)-one, 1.1 equivalents of methylamine were added and styrene was substituted for 4-methoxystyrene. The weight of product obtained from 10 gm starting halide was 6.5 gm after recrystallization from 75 ml of methoxyethanol. It melted at 243-246° C.

Example 13

Trans-6-[2-(4-methoxyphenyl)ethenyl]-2-[3-dimethylamino)-propyl]phthalazin-1(2H)-one hydrobromide

The procedure used was identical to that used in preparing 6-[2-(4-methocyphenyl)ethenyl]-2-[2-(dimethylamino)-ethyl]phthalazin-1(2H)-one (Example 3 above), except that 6-bromo-2-[3-dimethylamino)-propyl]phthalazin-1(2H)-one was substituted for 6-bromo-2-[2-dimethylamino)ethyl]phthalazin1(2H)-one. The weight of product obtained from 102 gm starting halide was 104 gm after recrystallization from 50 ml of methoxyethanol. It melted at 232-234° C.

Example 14

Trans-6-[2-(4-hydroxyphenyl)ethenyl]-2-[3-(dimethylamino)-propyl]phthalazin-1(2H)-one hydrochloride

Trans-6-[2-(4-methoxyphenyl)ethenyl]-2-[3-(dimethylamino)propyl]phthalazin-1(2H)-one (prepared as in Example 13) (46.6 gm, 0.122 mole) and 245 gm pyridine hydrochloride were heated together with stirring at 180° C for 7 hours. The hot melt was added to 1200 ml water and stirred over night. The product was filtered off and washed with three 400 ml portions of water. The damp product was slurried in 350 ml of tetrahydrofuran plus 350 ml methanol, acidified with gaseous HCl, filtered and washed with ethanol. The product, which weighed 36 gm, was recrystallized from 275 ml water. Wgt = 30.2 gm, mp = 282-283° C.

Example 15

Trans-6-[2-(4-hydroxyphenyl)ethenyl]-2-[2-(dimethylamino)-ethyl]-1(2H)-phthalazinone hydrochloride

Following the procedure of Example 1b, above trans-6-[2-(4-hydroxphenyl)ethenyl]-1(2H)-phthalazinone (19.8 g) and 2-(dimethylamino)ethyl chloride hydrochloride (17.3 g) were reacted to give the 2-[2-(dimethylamino)ethyl] derivative, which was converted to the hydrochloride (16 g), mp 283-285° C.

Example 16

Trans-2-[3-(dimethylamino)propyl]-6-(2-phenylethenyl)-1(2H)-phthalazinone

Step a

A mixture of 5-bromo-3-hydroxyphthalide (58 g), styrene (29 g), palladium acetate (0.25 g), tri-(o-tolyl)-phosphine (1.0 g), triethylamine (31 ml) and acetonitrile (75 ml), dimethylsulfoxide (75 ml) were heated with stirring at 95° C for 5 hrs. The reaction mixture was cooled and added to 500 ml of water. The product was extracted into ethyl acetate (200 ml) which was washed with water and dried over magnesium sulfate. Evaporation of the solvent gave 63 g of trans-3-hydroxy-5-(2-phenylethenyl)phthalide, mp 139-144° C.

Step b

The phthalide (12.7 g) was then reacted with N-[3-(dimethylamino)proply]hydrazine (8.4 g) according to the method of Example 17 below to give the 2-substituted phthalazinone (13 g), mp 132-133° C. The hydrochloride has a mp of 235-237° C.

Example 17

Trans-2-(2-hydroxyethyl)-6-(2-phenylethenyl)-1(2H)-phthalazinone

Trans-3-hydroxy-5-(2-phenylethenyl)phthalide (12.7 g) in isopropanol (75 ml) was heated to reflux and 2-hydroxyethylhydrazine (6 g) was added. After refluxing for 2 hours the reaction was cooled to 0°C and the precipitate as filtered and washed with isopropanol to give the 2-(2-hydroxyethyl)derivative (11 g), mp 154-156°C.

## Claims

1. Use of a 1(2H)-phthalazinone compound of the formula I

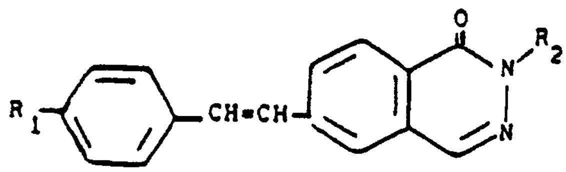

(I)

including all stereoisomeric forms and pharmaceutically aceptable addition salts thereof, wherein
$R_1$ is hydrogen, hydroxyl or $C_1$-$C_4$ alkoxy,
$R_2$ is hydrogen, $(CH_2)_mOH$,

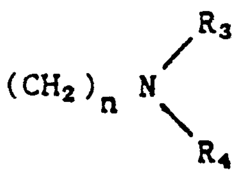 , 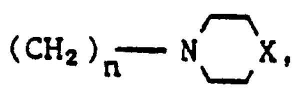 , 1-methyl-4-piperidinyl

or 1-pyrrolidinyl, $R_3$ and $R_4$ are independently hydrogen or $C_1$-$C_8$ alkyl,
X is $CH_2$ or $NR_5$,
$R_5$ is hydrogen or $C_1$-$C_8$ alkyl,
m is 2 or 3,
and n is 2 to 4,
provided that when m is 2, $R_1$ is hydrogen,
for the manufacture of a medicament for cytoprotective treatment in a mammal.

2. The use of Claim 1 wherein $R_1$ is hydrogen or methoxy, and $R_2$ is 3-(dimethylamino)propyl, 3-(diisopropylamino)propyl 2-(dimethylamino)ethyl, 4-(dimethylamino)butyl, 3-aminopropyl, 3-piperidinopropyl, 3-(1-piperazinyl)propyl, or 1-methyl-4-piperidinyl, and the stereoisomeric form is the trans isomer.

3. The use of Claim 1 in which the compound of formula I is trans-6-[2-(4-methoxyphenyl)ethenyl]-2-(diisopropylamino)ethyl]phthalazin-1(2H)-one hydrobromide.

4. The use of Claim 1 in which the compound of formula I is trans-6-[2-(4-methoxyphenyl)ethenyl]-2-(3-piperidinopropyl)phthalazin-1(2H)-one hydrobromide.

5. The use of Claim 1 in which the compound of formula I is trans-6-[2-(4-methoxyphenyl)ethenyl]-2-[2-(dimethylamino)ethyl]phthalazin-1(2H)-one hydrobromide.

6. The use of Claim 1 in which the compound of formula I is trans-6-[2-(4-methoxyphenyl)ethenyl]-2-[3-(1-piperazino)-propyl]phthalazin-1(2H)-one.

7. The use of Claim 1 in which the compound of formula I is trans-6-[2-(4-methoxyphenyl)ethenyl]-2-(1-methyl-4-piperidinyl)phthalazin-1(2H)-one.

8. The use of Claim 1 in which the compound of formula I is trans-6-[2-(4-methoxyphenyl)ethenyl]-2-[3-(methylamino)-propyl]phthalazin-1(2H)-one hydrobromide.

9. The use of Claim 1 in which the compound of formula I is trans-6-[2-(4-methoxyphenyl)ethenyl]-2-(3-aminopropyl)- 1(2H)-one hydrobromide.

10. The use of Claim 1 in which the compound of formula I is trans-6-[2-(4-methoxyphenyl)ethenyl]-2-[4-(dimethylamino)butyl]phthalazin-1(2H)-one bromide.

11. The use of Claim 1 in which the compound of formula I is cis-6-[2-(4-methoxyphenyl)ethenyl]-2-[3-(dimethylamino)-propyl]phthalazin-1(2H)-one cyclohexylsulfamate.

12. The use of Claim 1 in which the compound of formula I is trans-6-[2-(4-hydroxyphenyl)ethenyl]-phthalazin-1-(2H)-one.

13. The use of Claim 1 in which the compound of formula I is trans-6-[2-phenylethenyl]phthalazin-1(2H)-one.

14. The use of Claim 1 in which the compound of formula I is trans-6-[2-(4-methoxyphenyl)ethenyl]-2-[3-(dimethylamino)propyl]phthalazin-1(2H)-one hydrobromide.

15. The use of Claim 1 in which the compound of formula I is trans-6-[2-(4-hydroxyphenyl)ethenyl]-2-[3-(dimethylamino)propyl]phthalazin-1(2H)-one hydrochloride.

16. The use of Claim 1 in which the compound of formula I is trans-2-[3-(dimethylamino)propyl]-6-(2-phenylethenyl)-1(2H)-phthalazinone.

17. The use of Claim 1 in which the compound of formula I is trans-2-(2-hydroxyethyl)-6-(2-phenylethenyl)-1(2H)-phthalazinone.